# EUROPEAN PATENT APPLICATION

(11) **EP 0 684 477 A2**
(43) Date of publication of application: **29.11.1995**
(21) Application number: 95201336.5
(22) Date of filing: 22.05.1995
(51) Int. Cl.: G01N 33/96, G01N 33/92, G01N 33/94

(54) **Calibrator matrix**

(30) Priority: 26.05.1994 US 249856
(71) Applicant: BEHRING DIAGNOSTICS INC., Westwood, MA 02090-2399 (US)
(72) Inventor: Bahar, Izak, Sharon, MA 02067 (US); Inbar, Shai, Brookline, MA 02146 (US); Duggan, Dawn, Winchester, MA 01890 (US)
(74) Representative: Patentanwälte Dipl.-Ing. R. Splanemann Dr. B. Reitzner Dipl.-Ing. K. Baronetzky

(57) **Abstract**

This invention relates to an improved calibrator matrix for use in the calibration of tests, such as immunoassays or clinical chemistry tests, for analytes in serum and to the use of the calibrator for obtaining a more accurate calibration curve for use in such assay procedures.

## Description

This invention relates to an improved calibrator matrix for use in the calibration of tests, such as immunoassays or clinical chemistry tests, for analytes in serum and to the use of the calibrator for obtaining a more accurate calibration curve for use in such assay procedures.

Immunoassays and clinical chemistry tests are techniques used for the detection and quantification of specific components, i.e. analytes, in various media generally biological fluids. Chemistry tests for this purpose monitor detectable changes in the test medium resulting from chemical transformation of the analyte itself due to interaction with the test's reagents. Immunoassay methods are less direct in nature in the sense that they monitor detectable changes in the assay medium resulting from the binding of the analyte to a specific antibody. For this purpose, immunoassays employ antibodies specific to the analyte and a conjugate (or tracer), which is either an analyte, analyte analog or an antibody, bearing a moiety capable of generating a detectable signal. Various methodologies have been developed for performing immunoassays, employing different techniques by which the binding event results in a change in the detectable signal produced by the conjugate.

In order to convert the detectable signal from the assay to a quantitative result or concentration of the analyte, the assay must be calibrated. The calibration is done by running the assay first with a series of samples of predetermined concentrations. These samples are known as calibrators. The results obtained by reading the signals from the calibrators are used to generate, by using various curve fit models, a calibration curve spanning the entire assay range. The calibration curve can then be used to determine the concentration of an unknown sample from the signal it produces. When there are changes in either the calibrator or the test matrices over time, the calibrator often responds differently than a patient's serum to the assay, thus rendering the calibration ineffective.

Therefore it is an object of the present invention to provide a means for obtaining more reliable calibrators and calibration curves.

It has now been found that the addition of lipids such as, for example, cholesterol or a triglyceride preparation to a delipidated serum provides a calibrator which responds the changes in the assay reagent in a manner more similar to patient's sample.

More particularly, the matrix used for a calibrator presents certain problems. For example, for various reasons such as stability, availability and the risk of infection it is desirable to use a calibrator made from a non biological fluid, such as buffered water solution. However in many cases matrix effects may preclude such a possibility.

While in many cases their exact origin is not well understood, "matrix effects" are a well known and troublesome phenomena pertaining to biological assays. Biological fluids, such as serum, despite being predominantly water, include a complete mixture of various other components. Some of such components may exert influence on the assay such that the signal produced by a certain concentration of an analyte in one fluid, such as serum, may differ significantly form the signal produced by the same concentration of the analyte in a different medium, such as pure water. Matrix effects are often also seen between matrices more closely resembling each other, such as between human serum and sera of other species or even, to a lesser degree, between human sera from different individuals.

Matrix effects can result from a variety of reasons. For example differences in the physical properties of the calibrators and test matrices, such as viscosity and color, pH, ionic strength, salt concentration, protein concentration, and interfering substances are some factors which can cause matrix effects. Matrix effects are a potential source of problems in assays, in many ways, commonly affecting accuracy. Matrix effects seen between the calibrator matrix and patient samples can affect not only the accuracy but the shelf life of the assay.

Assay stability refers to the changes seen in the assay parameters over time. Assay shelf life refers to the period in which the assay still fulfills the requirement to provide accurate test results for its specific use. The chemical instability of the assay reagents causes changes over time in the level of the signal produced by the assay for a certain level of analyte. Such changes in the assay can be seen in many cases over a relatively short period of time. This does not mean, however, that the assay has outlived its usefulness as those changes can be compensated for, i.e. "calibrated out", by recalibrating the assay for a certain period of time.

Calibrating out the changes in the assay reagents can be successful only if both the calibrator and the test matrices respond to the changes in the reagents in an identical manner. When the matrix employed for the calibrators is not the same as the patients or test matrix, the matrix effects may result in a significant shortening of the shelf life of the assay.

In many assay methods, the sample is either pretreated prior to use or is largely diluted in water or buffer solution. In such cases the initial differences between the calibrator matrix and the sample matrix become insignificant. However pretreatment or predilution is either manual and labor intensive, or time consuming for an automated system, limiting throughput. Therefore assay methods have been developed in which pretreatment or predilution is eliminated or minimized. For such assays, matrix effects are expressed at a maximum and the use of a calibrator's matrix that is as close as possible to the samples' medium becomes imperative.

For assays intended to be used to determine the concentration of analyte in a non diluted human serum, the ideal calibrator matrix is unprocessed human serum. A problem involved with using such a matrix is its stability, especially with the tendency of lipids emulsified in the serum to separate over time, a process known as "shedding", which results in an opaque milky appearance. The shedding is known to affect assay results and accordingly limits the useful shelf life of calibrators made in such a matrix. To avoid the problems associated with shedding, various methods have been developed to remove the lipid components from the serum pool intended to be used for calibrators, a process called "lipid stripping". While lipid stripped serum resembles human serum to a large degree, there are matrix effects between whole and lipid stripped sera. The effects are dependent on various factors, for example, an analyte may exhibit an affinity of binding to lipids, or the serum lipids may affect the level of non specific binding in the assay.

Non specific binding is a general problem encountered in immunoassays, especially heterogeneous assay employing a solid phase. In most common types of immunoassays, such as the competitive binding assay and the two sites sandwich ELISA, the separation of free and bound conjugate is necessary as part of the assay protocol. This step is usually called the "wash" step because it involves washing away the unbound conjugate. In a typical wash, when the antibody used in the assay, or in some cases, the analyte, is immobilized on a solid phase the wash is facilitated. However a fraction of the conjugate can bind non specifically to the solid phase material thus giving rise to a false signal. This effect of the false signal is minimized when the non specific binding in the calibrator and the patients' samples remain consistent. If the level of nonspecific binding in the calibrator's matrix and patient samples changes to different degrees over time in response to changes in the assay reagents, such changes result in the inability to calibrate out age effects and limit the useful shelf life of the assay reagents.

One example is an immunoassay of digoxin, a competitive ELISA where the signal obtained is inversely proportional to the dose of analyte measured. A monoclonal antibody to digoxin is immobilized non-covalently on a glass fiber matrix. It is treated with digoxin-alkaline phosphatase conjugate and then washed with a fluorogenic substrate solution. The fluorogenic substrate is used for both wash and development of the assay signal. There is a level of non specific binding of conjugate to the fiber matrix and this contributes to the assay signal.

It has been found that there were changes in the level of non specific binding of the antigen-alkaline phosphatase conjugate after storage of about 6 months or more, even if stored at 4°C. These changes in the binding were different when the assay was run with delipidated calibrators or with patients samples., causing differences in the results, despite recalibration. It has also been found that the instability can be accelerated by placing the conjugate at 37°C for 4-7 days. Such an acceleration of the instability was used to facilitate a study of the problem.

It has been found that the addition of certain components of serum lipid fraction such as, for example, cholesterol or a triglyceride preparation, to the delipidated serum provides a matrix which responds to the changes in the assay reagent in a manner more similar to patients samples. The addition of the cholesterol or triglyceride preparations does not alter the long term stability of the calibrators and no shedding of the lipid components was observed. Thus the matrix effects have been minimized without sacrificing stability of the calibrators, significantly extending the shelf life of the assay. Use of the calibrator even with components which have been stored for over six months gives reliable results.

A calibrator matrix is generally prepared from delipidated human serum which is commercially available. A series of solutions are then prepared containing a range of concentrations of the component which is measured in the assay.

The cholesterol or triglyceride is added at a wide range of levels, preferably at about physiological concentrations, more preferably from about 150 to 250 mg/dl, most preferably at about 200 mg/dl.

The present invention includes the calibrator to which the lipid such as cholesterol or triglyceride has been added, as described above. It also includes the use of the calibrator in carrying out the assay.

Additional aspects of the invention are set forth in the non-limting examples recorded below.

### Example 1

### Recovery of serum samples in OPUS® Digoxin Immunoassay

The Digoxin assay in serum was designed to run on OPUS® Immunoassay System marketed by Behring Diagnostics, Inc. The assay is based on the principle of the competitive immunoassay and was run in a sequential mode (back titration). The assay was performed in three separate steps, all executed automatically by the instrument. In the first step, the instrument applied 30·l of serum sample onto the fiber (glass fiber matrix GF/F, Whatman). The sample was allowed to incubate for four minutes and the digoxin in the sample bound to the antibody on the glass fibers. Then 20·l of digoxin-alkaline phosphatase conjugate solution was applied and the sample was incubated for 1 minute. Then 75·l of substrate (4-methyl-umbelliferyl phosphate solution) was dispensed into the well of the wash port on the side of the test module. The solution, through capillary action, washed the unbound conjugate fraction toward the other end of the glass fiber into an absorbing pad, leaving the bound fraction in the center. The intensity of the fluorescence generated by methyl unbelliferone was measured and converted to amount of digoxin by the instrument using a stored calibration curve generated from a delipidated calibrator matrix in the instrument memory.

The results are set forth in Table I.

**Table 1**

| Sample recovery with fresh modules | |
|---|---|
| Sample | Recovery, ng/ml |
| Sample 1 | 0.71 |
| Sample 2 | 2.01 |
| Sample 3 | 3.00 |
| Sample 4 | 3.07 |

The recoveries of the samples are appropriate for the assay range.

### Example 2

### Recovery with 1.5 month old and 6 month modules

The assay was carried out as described in Example 1 except that modules 1.5 months old and 6.0 months old were used in the assay. The results are set forth in Table 2.

**TABLE 2**

| Sample recovery with old modules | | | |
|---|---|---|---|
| Sample | 1.5 months | 6 months Lot 1 | 6 months Lot 2 |
| Sample 1 | 2.94 | 3.69 | 3.22 |
| Sample 2 | 3.00 | >4.0 | 3.95 |

It can be seen from the table that at a period of 6 months of storage of the modules at 4°C, the assay recovers the Sample 1 and 2 serum as high as .4.0 ng/ml instead of the appropriate 3.0 ng/ml. The assay is no longer functional in the region between 3 and 4 ng/ml.

### Example 3

### Recovery with conjugate stored at elevated temperature

The assay was carried as described in Example 1 except that modules which had been stored at 37°C for 4 days were used. The results are set forth in Table 3.

**Table 3**

| Sample recovery with conjugate stored at 37°C | | |
|---|---|---|
| Level | 4°C | 37°C |
| Sample 1 | 2.93 | >4.0 |
| Sample 2 | 3.07 | >4.0 |

### Example 4

### Sample recovery using old modules and cholesterol supplemented calibrators

The assay was carried out as described in Example 2 except that cholesterol (PENTEX® CHOLESTEROL SUPERTRATE, Miles, Inc, Diagnostics Division, 195 West Birch Street, Kankakee, Illinois 60901) was added to the delipidated calibrator serum at a concentration of 200 mg/dl. The results are set forth in Table 4.

**Table 4**

| Sample recovery using old modules and cholesterol supplemented calibrators | | | |
|---|---|---|---|
| Sample | 1.5 months | 6 months Lot 1 | 6 months Lot 2 |
| Sample 1 | 2.57 | 2.76 | 2.85 |
| Sample 2 | 3.08 | 3.14 | 3.20 |

### Example 5

### Sample recovery with conjugate stored at elevated temperature and cholesterol supplemented calibrator

The assay was carried out as described in Example 3 except that cholesterol (200 mg/dl) was added to the delipidated calibrator serum. The results are set forth in Table 5.

**Table 5**

| Sample recovery with conjugate stored at 37°C and cholesterol supplemented calibrators | | |
|---|---|---|
| Sample | 4°C | 37°C |
| Sample 1 | 2.73 | 2.6 |
| Sample 2 | 3.00 | 3.05 |

### Example 6

### Sample recovery with conjugate stored at elevated temperature and triglyceride supplemented calibrators

The assay was carried out as described in Example 5 except that the calibrator was supplemented with triglyceride (PENTEX® TRIGLYCERIDE SUPER-TRATE, Miles, Inc, Diagnostics Division, 195 West Birch Street, Kankakee, Illinois 60901) in place of cholesterol. The results are shown in Table 6.

**Table 6**

| Sample recovery with conjugate stored at 37°C and triglyceride supplemented calibrators | | |
|---|---|---|
| Sample | 4°C | 37°C |
| Sample 1 | 2.62 | 2.59 |
| Sample 2 | 2.91 | 3.06 |

While only certain preferred features of the invention have been shown by way of illustration, may modifications and changes will occur to those skilled in the art. It is, therefore, to be understood that the appended claims are intended to cover all such modifications and changes as fall within the true spirit of the invention.

## Claims

1. A method for reducing matrix effects arising from the use of lipid stripped serum as a matrix for calibrators in assays for analytes in serum which comprises adding a lipid preparation to the lipid stripped matrix.

2. The method of Claim 1 wherein the lipid preparation is cholesterol.

3. The method of Claim 1 wherein the lipid preparation is triglyceride.

4. The method of Claim 1 wherein the assay is an immunoassay.

5. The method of Claim 1 wherein the assay is an immunoassay used to detect the presence of digoxin.

6. The method of Claim 5 wherein the lipid preparation is added at a physiological concentration.

7. The method of Claim 6 wherein the lipid preparation is added at a concentration of from about 150 to 250 mg/dl.

8. The method of Claim 7 wherein the lipid preparation is added at a concentration of about 200 mg/dl.

9. The method of Claim 8 wherein the lipid preparation is cholesterol.

10. The method of Claim 8 wherein the lipid preparation is triglyceride.

11. A calibrator serum for use in reducing matrix effects arising from the use of lipid stripped serum which comprises a lipid stripped calibrator serum and a lipid preparation.

12. The calibrator serum of Claim 11 which further contains a known amount of digoxin.

13. The calibrator serum of Claim 12 wherein the lipid preparation is cholesterol.

14. The calibrator serum of Claim 12 wherein the lipid preparation is triglyceride.

15. The calibrator serum of Claim 11 wherein the lipid preparation is present at a physiological concentration.

16. The calibrator serum of Claim 15 wherein the lipid preparation is present at a concentration of from about 150 mg/dl to about 250 mg/dl.

17. The calibrator serum of Claim 16 wherein the lipid preparation is present at a concentration of about 200 mg/dl.

18. The calibrator serum of Claim 17 wherein the lipid preparation is cholesterol.

19. The calibrator serum of Claim 17 wherein the lipid preparation is triglyceride.
